Europäisches Patentamt

European Patent Office

Office européen des brevets

(11) Publication number : **0 622 073 A1**

# (12) EUROPEAN PATENT APPLICATION

(21) Application number : **94302943.9**

(22) Date of filing : **25.04.94**

(51) Int. Cl.[5] : **A61K 9/48**

(30) Priority : **26.04.93 JP 99593/93**

(43) Date of publication of application :
**02.11.94 Bulletin 94/44**

(84) Designated Contracting States :
**AT BE CH DE DK ES FR GB GR IE IT LI LU NL PT SE**

(71) Applicant : **TAKEDA CHEMICAL INDUSTRIES, LTD.**
**1-1, Doshomachi 4-chome**
**Chuo-ku, Osaka 541 (JP)**

(72) Inventor : **Marunaka, Shigeyuki**
**19-3-403, Aikawa 2-chome,**
**Higashiyodogawa-ku**
**Osaka 533 (JP)**
Inventor : **Kikuta, Junichi**
**23-A11-202, Yamadanishi 1-chome,**
**Suita**
**Osaka 565 (JP)**
Inventor : **Makino, Tadashi**
**12-39-1, Mishimaoka 2-chome,**
**Ibaraki**
**Osaka 567 (JP)**

(74) Representative : **Laredo, Jack Joseph**
**Elkington and Fife**
**Prospect House**
**8 Pembroke Road**
**Sevenoaks, Kent TN13 1XR (GB)**

(54) **Easier to swallow hard capsules.**

(57)  Hard capsules having a surface coating of a physiologically acceptable water repellent has an improved swallowability via reduced adhesion to the throat and esophagus upon intake.

EP 0 622 073 A1

The present invention relates to hard capsules with improved swallowability.

Hard capsules, a widely used dosage form of solid preparations, can adhere to the throat and esophagus upon intake and disintegrate there, causing premature release of ingredient chemicals and hence ulceration [Clin. Pharmac., 18, 250 (1984); Clin. Pharmacol. Ther., Jan., 72 (1985); British Medical Journal, 1, 1673 (1979)]. No attempts have been made to solve this problem.

The present inventors investigated the solution to this problem, and found that hard capsule adhesion to the throat and esophagus upon intake is due to the high water solubility and hence high adhesion of gelatin, the major component of the capsules, and that the solution is to improve capsule swallowability through throat and esophagus by coating the hard capsule surface with a water repellent to such extent that the capsule's rapid disintegratability is not affected.

Accordingly, the present invention relates to hard capsules having a surface coating of a physiologically acceptable water repellent.

In the present invention, the physiologically acceptable water repellent is defined as having water solubility not higher than 0.03 g/l at 25°C. Such water repellents include aliphatic saturated hydrocarbons having 15 to 55 carbon atoms, such as paraffin (liquid paraffin, solid paraffin) and microcrystalline wax, oils and fats having a melting point of 60 to 90°C, such as carnauba wax, hardened oils, bleached beeswax and white wax, silicones having a viscosity of 95 to 1,050 cs, at 25°C such as silicone oil and silicone resin, fatty acids such as stearic acid, esters of fatty acid such as sucrose ester of fatty acid, glycerol ester of fatty acid and polyglycerol ester of fatty acid, polyhydric alcohols having a melting point of 4 to 65°C, such as stearyl alcohol and cetanol, and film base polymers such as shellac and ethyl cellulose. Paraffin, carnauba wax, white wax, silicone, stearic acid, stearyl alcohol and shellac are preferred.

Hard capsule coating is achieved by first thermally fusing a physiologically acceptable water repellent (hereinafter also referred to as coating agent) or dissolving it in an organic solvent to prepare a coating solution, then applying the coating solution to the hard capsules by a conventional method using a coater. Thermal fusion of the water repellent is then achieved by heating it to a temperature exceeding its melting point. The organic solvent used to dissolve the water repellent is exemplified by normal hexane, methylene chloride and alcohols. Although the water repellent is not subject to limitation as to concentration, it is preferable to use it at 5% by weight to saturation concentration, with preference given to concentrations near saturation concentration. Too low concentrations are undesirable for practical use because drying takes longer. Although any conventional coater can be used, the HICORTER (Freund Industrial Co., Ltd.), the ACCELA COATER (Manesty), the DRIACOATER (Powrex Corporation), the sugar coating pan (Kikusui Seisakusho), the VG Coater (Kikusui Seisakusho) etc. are preferable.

The coating solution is used in amounts equivalent to coating agent ratios of 0.1 to 10.0% by weight, preferably 0.5 to 1.5% by weight per capsule base. The thickness of the coating agent layer formed on the hard capsule surface is preferably 0.1 to 100 μ, more preferably 1 to 50 μ. Too thick a coating agent layer is undesirable because capsule disintegration is delayed. Too thin an agent layer hampers the desired effect of the present invention.

In addition to the above-described water repellent, the coating solution may incorporate coating aids (e.g., polyethylene glycol, glycerol, polysorbate 80), taste correctives (e.g., sucrose, lactose, mannitol, maltitol, sorbitol, xylitol, aspartame, stevia, saccharin), odor correctives (e.g., menthol, peppermint oil, lemon oil, banana oil) and coloring agents (e.g., tar-based food dyes, aluminum lake, iron oxide red, titanium oxide). These coating aids are added to the coating solution in an proportion ranging from 0.1 to 10 weight %.

The thus-obtained hard capsules of the present invention are orally used as packed with pharmaceutical powder, granules, semi-solids, liquids etc., as with conventional hard capsules.

According to the present invention, hard capsule swallowability is improved by preventing capsule adhesion to the throat and esophagus upon intake.


[Examples]


The present invention is hereinafter described in more detail by means of the following examples, in which capsule swallowability and disintegratability were evaluated:

1) Swallowability (sensory evaluation)

Each capsule was ingested without water and throat adhesion and residual sensation were evaluated by 5 panelists.

1: Swallowing easy, without adhesion

2: Slight residual sensation, though passage is easy, without adhesion

3: Slight adhesion and residual sensation

4: Swallowing difficult due to adhesion and residual sensation

2) Disintegratability (JP XII)

Time from ingredient granule release start to release completion upon capsule disintegration was determined using a disintegration tester (in water at 37°C, disc used).

Example 1

500 g of granule-packed No. 1 capsules (about 1,119 cp, 447 mg/cp, empty capsules consisting of 85% by weight gelatin and 15% by weight water) were placed in a sugar-coating pan (12 inches), and various coating solutions were added while the pan was rotated at 40 rpm to coat the capsules, followed by drying at 40°C, to yield samples.

| Comparative sample | | |
|---|---|---|
|    Packed hard capsules | 447.00 mg | |
|    (empty capsules | 77.00 mg) | |
| Inventive sample 1 | | |
|    Packed hard capsules | 447.00 mg | |
|    (empty capsules | 77.00 mg) | |
| Coating Solution Composition | Weight | Ratio of Empty Capsules |
|    Shellac | 0.46 mg | 0.60 W/W% |
|    Castor oil | 0.12 mg | 0.16 W/W% |
|    Carnauba wax | 0.52 mg | 0.68 W/W% |
|    (99% ethanol | 4.2 μl) | |
|    (n-hexane | 3.1 μl) | |
|                  Total | | 1.44 W/W% |

Table 1

| | Swallowability | Disintegratability |
|---|---|---|
| Inventive sample 1 | 1 | 1.5 to 3.3 minutes |
| Comparative sample | 4 | 2.0 to 3.5 minutes |

The inventive sample showed no disintegratability change or adhesion or residual sensation upon intake, demonstrating improved swallowability, while the comparative sample showed adhesion and residual sensation.

Example 2

500 g of granule-packed No. 1 capsules (about 1,119 cp, 447 mg/cp, empty capsules consisting of 85% by weight gelatin and 15% by weight water) were placed in a High Coater (HCT-20), and various coating solutions were sprayed while the coater was rotated at 20 rpm to coat the capsules, followed by drying at 40°C, to yield samples.

| Inventive sample 2 | | |
|---|---|---|
|    Packed hard capsules | 447.00 mg | |
|    (empty capsules | 77.00 mg) | |
| Coating Solution Composition | Weight | Ratio of Empty Capsules |
| Carnauba wax | 0.38 mg | 0.49 W/W% |
| White wax | 0.04 mg | 0.05 W/W% |
| (n-hexane | 8.4 μl) | |
| Total | | 0.54 W/W% |

Table 2

| | Swallowability | Disintegratability |
|---|---|---|
| Inventive sample 2 | 2 | 2.0 to 3.5 minutes |
| Comparative sample | 4 | 2.0 to 3.5 minutes |

The inventive sample showed a swallowability improving tendency.

Example 3

500 g of granule-packed No. 2 capsules (about 2,000 cp, 255 mg/cp, empty capsules consisting of 85% by weight gelatin and 15% by weight water) were placed in a High Coater (HCT-20), and various coating solutions were added while the coater was rotated at 20 rpm to coat the capsules, followed by drying at 40°C, to yield samples.

| Inventive sample 3 | | |
|---|---|---|
|    Packed hard capsules | 255.00 mg | |
|    (empty capsules | 65.00 mg) | |
| Coating Solution Composition | Weight | Ratio to Empty Capsules |
|    Silicone oil | 1.79 mg | 2.75 W/W% |
|     (KS66) | | |

Table 3

| | Swallowability | Disintegratability |
|---|---|---|
| Inventive sample 3 | 2 | 2.2 to 3.2 minutes |
| Comparative sample | 4 | 2.0 to 3.5 minutes |

The inventive sample showed a swallowability improving tendency.

Example 4

500 g of granule-packed No. 1 capsules (about 1,119 cp, 447 mg/cp, empty capsules consisting of 85% by weight gelatin and 15% by weight water) were placed in a Doria Coater (DC2500), and various coating solutions were sprayed while the coater was rotated at 40 rpm to coat the capsules, followed by drying at 40°C, to yield samples.

Inventive sample 4

| | Weight | Ratio to Empty Capsules |
|---|---|---|
| Packed hard capsules | 447.00 mg | |
| (empty capsules | 77.00 mg) | |
| Coating Solution Composition | Weight | Ratio to Empty Capsules |
| Carnauba wax | 0.20 mg | 0.26 W/W% |
| White wax | 0.03 mg | 0.04 W/W% |
| Silicone oil (KS66) | 4.47 mg | 5.81 W/W% |
| (n-hexane | 4.4 µl) | |
| Total | | 6.11 W/W% |

| Inventive sample 5 | | |
|---|---|---|
| Packed hard capsules | 447.00 mg | |
| (empty capsules | 77.00 mg) | |
| Coating Solution Composition | Weight | Ratio of Empty Capsules |
| Carnauba was | 0.20 mg | 0.26 W/W% |
| White wax | 0.03 mg | 0.04 W/W% |
| Silicone oil (KS66) | 0.89 mg | 1.15 W/W% |
| (n-hexane | 4.4 µl) | |
| Total | | 1.45 W/W% |

| Inventive sample 6 | | |
|---|---|---|
| Packed hard capsules | 447.00 mg | |
| (empty capsules | 77.00 mg) | |
| Coating Solution Composition | Weight | Ratio of Empty Capsules |
| Carnauba wax | 0.40 mg | 0.52 W/W% |
| White wax | 0.06 mg | 0.08 W/W% |
| Silicone oil (KS66) | 1.79 mg | 2.32 W/W% |
| (n-hexane | 8.8 µl) | |
| Total | | 2.92 W/W% |

Table 4

|  | Swallowability | Disintegratability |
|---|---|---|
| Inventive sample 4 | 2 | - |
| Inventive sample 5 | 2 | - |
| Inventive sample 6 | 2 | 1.8 to 3.0 minutes |
| Comparative sample | 4 | 2.0 to 3.5 minutes |

The inventive samples showed a swallowability improving tendency, while the comparative sample showed adhesion and residual sensation after intake.

Example 5

500 g of empty No. 1 capsules (about 6,500 cp, empty capsules consisting of 85% by weight gelatin and 15% by weight water) were placed in a VG Coater, and various coating solutions were sprayed under reduced pressure (about 100 mmHg), followed by drying at 40°C, to yield samples.

| Inventive sample 7 | | |
|---|---|---|
| Empty hard capsules | 77.00 mg | |
| Coating Solution Composition | Weight | Ratio of Empty Capsules |
| Solid paraffin | 0.89 mg | 1.16 W/W% |
| (n-hexane | 89.0 μl) | |

Table 5

|  | Swallowability | Disintegratability |
|---|---|---|
| Inventive sample 7 | 1 | 1.5 to 3.6 minutes |
| Comparative sample | 5 | 2.0 to 3.5 minutes |

The inventive sample showed an improved swallowability without loss of disintegratability.

Example 6

500 g of granule-packed No. 1 capsules (about 1,119 cp, 447 mg/cp, empty capsules consisting of 85% by weight gelatin and 15% by weight water) were placed in a sugar-coating pan, and various coating solutions were added while the pan was rotated at 40 rpm to coat the capsules, followed by drying at 40°C, to yield samples.

| Inventive sample 8 | | |
|---|---|---|
| Packed hard capsules | 447.00 mg | |
| (empty capsules | 77.00 mg) | |
| Coating Solution Composition | Weight | Ratio to Empty Capsules |
| Carnauba wax | 0.20 mg | 0.26 W/W% |
| White wax | 0.03 mg | 0.04 W/W% |
| Solid paraffin | 0.45 mg | 0.58 W/W% |
| (n-hexane | 49.4 μl) | |
| Total | | 0.88 W/W% |

| Inventive sample 9 | | |
|---|---|---|
| Packed hard capsules | 447.00 mg | |
| (empty capsules | 77.00 mg) | |
| Coating Solution Composition | Weight | Ratio of Empty Capsules |
| Carnauba wax | 0.20 mg | 0.26 W/W% |
| White wax | 0.03 mg | 0.04 W/W% |
| Solid paraffin | 0.89 mg | 1.16 W/W% |
| (n-hexane | 93.4 μl) | |
| Total | | 1.46 W/W% |

Table 6

| | Swallowability | Disintegratability |
|---|---|---|
| Inventive sample 8 | 1 | - |
| Inventive sample 9 | 1 | 1.5 to 2.8 minutes |
| Comparative sample | 4 | 2.0 to 3.5 minutes |

The inventive samples showed an improved swallowability without loss of disintegratability.

All the above inventive samples showed an improved swallowability; inventive samples 7, 8 and 9, in particular, showed excellent swallowability.

A storage test gave good results, in which capsules showed no mutual adhesion in 4-week storage at 60°C.

Example 7

500 g of granule-packed No. 1 capsules (about 1,119 cp, 447 mg/cp, empty capsules consisting of 85% by weight gelatin and 15% by weight water) were placed in a High Coater (HCT-20), and various coating solutions were sprayed while the coater was rotated at 20 rpm to coat the capsules, followed by drying at 40°C, to yield samples.

| Inventive sample 2 | | |
|---|---|---|
| Packed hard capsules | 447.00 mg | |
| (empty capsules | 77.00 mg) | |
| Coating Solution Composition | Weight | Ratio of Empty Capsules |
| Carnauba was | 0.42 mg | 0.54 W/W% |
| (n-hexane | 8.4 μl) | |
| Total | | 0.54 W/W% |

Table 7

| | Swallowability | Disintegratability |
|---|---|---|
| Inventive sample 9 | 2 | 2.0 to 4.0 minutes |
| Comparative sample | 4 | 2.0 to 3.5 minutes |

The inventive sample showed a swallowability improving tendency.

## Claims

1. A hard capsule having a surface coating of a physiologically acceptable water repellant.

2. The hard capsule of claim 1, wherein said physiologically acceptable water repellent is used at 0.1 to 10% by weight of the capsule base.

3. The hard capsule of claim 1, wherein the thickness of the surface coating of said physiologically acceptable water repellent is 0.1 to 100 μ.

4. The hard capsule of claim 1, 2 or 3, wherein said physiologically acceptable water repellent has a water solubility of not higher than 0.03 g/l at 25°C.

5. The hard capsule of claim 1, wherein the water repellant is one or more members selected from the group consisting of ① an aliphatic saturated hydrocarbon having 15 to 55 carbon atoms, ② an oil and fat having a melting point of 60 to 90°C, ③ a silicone having a viscosity of 95 to 1,050cs at 25°C, ④ a fatty acid, ⑤ an ester of fatty acid, ⑥ a polyhydric alcohol having a melting point of 4 to 65°C and ⑦ a film base polymer.

6. The hard capsule of claim 1, wherein the water repellant is one or more members selected from the group consisting of paraffin, carnauba wax, silicone, stearic acid, stearyl alcohol, white wax and shellac.

7. The hard capsule of claim 1, wherein the water repellant is one or more members selected from the group consisting of solid paraffin, carnauba wax, silicone oil, white wax and shellac.

**European Patent Office**

# EUROPEAN SEARCH REPORT

Application Number

EP 94 30 2943

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | FR-A-2 216 985 (LILLY INDUSTRIES LIMITED)<br>* claims 1,2,4 *<br>* page 2, line 24 - line 28 *<br>* page 3, line 26 - line 37 *<br>--- | 1-7 | A61K9/48 |
| X | US-A-2 512 192 (ERNEST C. YEN ET AL.)<br>* claim 2 *<br>* column 2, line 41 - column 4, line 46 *<br>--- | 1,2,4-7 | |
| X | DE-A-17 67 425 (R. P. SCHERER GMBH)<br>* claim 1 *<br>* page 3, line 7 - line 30 *<br>* page 6, paragraph 2 *<br>----- | 1,2,4-7 | |
| | | | TECHNICAL FIELDS SEARCHED (Int.Cl.5)<br><br>A61K |

The present search report has been drawn up for all claims

| Place of search | Date of completion of the search | Examiner |
|---|---|---|
| THE HAGUE | 8 July 1994 | Ventura Amat, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)